# EUROPEAN PATENT APPLICATION

(11) **EP 4 512 930 A1**
(43) Date of publication of application: **26.02.2025**
(21) Application number: 23208692.6
(22) Date of filing: 09.11.2023
(51) Int. Cl.: C25B 1/04, C25B 11/052, C25B 11/073, C02F 1/461, C02F 3/00, C12M 1/107, C12P 3/00

(54) **MICROBIAL ELECTROLYSIS CELL SUPPRESSING METHANE GENERATION AND METHOD OF PRODUCING HYDROGEN USING SAME**

(30) Priority: 25.08.2023 KR 20230111780
(71) Applicant: EN Corporation, Cheonan-si Chungcheongnam-do 31075 (KR)
(72) Inventor: JEON, Yong Won, 34162 Daejeon (KR); KIM, Jun Hyun, 12710 Gwangju-si, Gyeonggi-do (KR)
(74) Representative: Isarpatent

(57) **Abstract**

Disclosed are a microbial electrolysis cell suppressing methane generation and a method of producing hydrogen using the same, and more particularly microbial electrolysis cell technology, which prevents the growth of methanogens inside a reactor during operation of a microbial electrolysis cell by aerating a substrate for use in a microbial electrolysis cell with acetylene gas before supply of the substrate, thereby suppressing consumption of the hydrogen and substrate by methanogens, ultimately increasing the hydrogen yield and lifespan of the microbial electrolysis cell.

## Description

### BACKGROUND OF THE INVENTION

### Field of the Invention

The present invention relates to a microbial electrolysis cell suppressing methane generation and a method of producing hydrogen using the same, and more particularly to microbial electrolysis cell technology, which prevents the growth of methanogens inside a reactor during operation of a microbial electrolysis cell, thereby suppressing consumption of the hydrogen and substrate by methanogens, ultimately increasing the hydrogen yield and lifespan of the microbial electrolysis cell.

### Description of the Related Art

Techniques for reusing renewable resources are receiving attention due to decreased fossil fuel reserves, and in particular, eco-friendly and highly efficient energy sources are being developed around the world to solve the problem of environmental pollution. Thereamong, hydrogen energy has great potential as a future energy source because it minimizes the generation of greenhouse gases that affect climate change.

Electrical energy is usually required to produce hydrogen, but since most electrical energy is a product of fossil fuels, microbial electrolysis cell (MEC) technology is in the spotlight to minimize the energy required for hydrogen production.

Under standard conditions (single-chamber type, 50 mM phosphate buffer solution), microbial electrolysis cells may generate methane by reducing carbon dioxide at a cathode, but the overpotential of the reaction is very high and hydrogen is generated before methane, and thus the main cause of methane gas generation is regarded as microorganisms, rather than reduction reaction at the electrode.

Microbial electrolysis cells generally use wastewater as an inoculum, and various microorganisms including methanogens usually exist in wastewater. Methane may be generated when acetate, which is used as a substrate, is hydrolyzed by methanogens or when hydrogen produced at the cathode and carbon dioxide generated by degrading the substrate at the anode are converted into methane by methanogens.

As such, when the substrate is not degraded well due to slow reaction of the microbial electrolysis cell or when the produced hydrogen is discharged slowly to the outside of the battery, methane may be generated from the remaining substrate or non-discharged hydrogen using methanogens that are introduced during inoculation, which is undesirable.

In a microbial electrolysis cell, methanogens compete with and consume substrates with electroactive microorganisms at the anode, so that the amount of hydrogen produced may decrease due to a decrease in the amount of electrons to be sent to the cathode, and methane may also be generated by consuming the produced hydrogen, deteriorating the ability of the microbial electrolysis cell to produce hydrogen. Moreover, methane is a material used as fuel, but it is one of the major greenhouse gases and may cause environmental pollution by emitting carbon dioxide when burned. Since the purpose of introducing a microbial electrolysis cell is not only to treat wastewater but also to produce hydrogen, which is an eco-friendly energy source, suppressing methane generation in the microbial electrolysis cell is necessary to improve the environment and hydrogen production efficiency.

Korean Patent No. 10-1714431 (related art) discloses a microbial electrolysis cell and a method of producing hydrogen using the same. In particular, in order to minimize methane generation in the microbial electrolysis cell, applying external voltage is performed in the presence of an external resistance of 10 to 30 Ω.

Here, the microbial electrolysis cell uses low electrical energy compared to water electrolysis hydrogen production technology. However, if external electrical energy is used to suppress methane generation, it is against the purpose of introducing the microbial electrolysis cell, and thus other methods capable of suppressing methane generation are needed.

### [Citation List]

### [Patent Literature]

Korean Patent No. 10-1714431 (March 01, 2017)

### SUMMARY OF THE INVENTION

An object of the present invention is to provide a method of minimizing methane generation in a microbial electrolysis cell.

In order to accomplish the above object, the present invention provides a method of producing hydrogen using a microbial electrolysis cell.

In an embodiment, the method of producing hydrogen may include forming an electroactive biofilm on an anode, aerating a liquid substrate with a mixed gas of acetylene gas and nitrogen at a predetermined ratio to dissolve acetylene gas in the substrate, supplying the substrate in which acetylene gas is dissolved, and producing hydrogen at a cathode.

In an embodiment, the liquid substrate may be obtained by treating wastewater with phosphoric acid, and the wastewater may be activated sludge in a sewage treatment plant or wastewater discharged from any one selected from among a distillery house that produces soju or whiskey, a winery house, and a brewery house.

In an embodiment, the mixed gas may be a mixture of acetylene gas and nitrogen at a mixing ratio (w/w) of 1:99 to 3:97.

In an embodiment, the substrate may be aerated with the mixed gas for 5 minutes to 15 minutes.

In another embodiment, the present invention provides a microbial electrolysis cell used in the method of producing hydrogen described above.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a graph showing the current generation pattern after inoculation of a microbial electrolysis cell with microorganisms;
FIG. 2 is a graph comparing the amounts of gases generated in the microbial electrolysis cell with or without pretreatment with acetylene gas; and
FIG. 3 is a graph comparing the proportions of gases generated in the microbial electrolysis cell with or without pretreatment with acetylene gas.

### DETAILED DESCRIPTION OF THE INVENTION

Hereinafter, some embodiments of the present invention will be described in detail using the drawings, but are not construed as limiting the present invention, and all transformations, equivalents, and substitutions including the technical spirit of the present invention should be understood to be included in the scope of the present invention.

In this specification, singular expressions include plural expressions unless the context clearly dictates otherwise.

In this specification, when any one component is described as "having" or "comprising" a sub-component, it means that other components may be further included, rather than excluding other components, unless specifically stated to the contrary.

### 1. Configuration of microbial electrolysis cell (MEC)

A box-type PVC reactor having a width of 35 cm, a length of 65 cm, and a height of 5 cm was provided with 23 carbon fiber brush anodes having a diameter of 3 cm and a length of 35 cm that were connected in parallel and with one cathode made of a 60-mesh stainless steel net having a width of 35 cm and a length of 60 cm.

### 2. Formation of electroactive biofilm

In order to form an electroactive biofilm on the anode of a microbial electrolysis cell, a substrate in which 10% activated sludge in a sewage treatment plant was mixed with 50 mM phosphate buffer solution (PBS, pH 7) with 1 g/L sodium acetate was supplied, and the current generation was measured by applying an external voltage of 0.8 V. Then, the substrate was replaced every two days until the generated current gradually increased and reached a constant level.

The activated sludge used for the substrate is only an example, or alternatively, wastewater discharged from any one selected from among a distillery house that produces soju or whiskey, a winery house, and a brewery house may be used.

### 3. Pretreatment of substrate using acetylene gas

A prepared liquid substrate was aerated with a mixed gas of 2-3% acetylene gas and 97-98% nitrogen (i.e., the mixing ratio of acetylene gas to nitrogen is 2:98 to 3:97 (w/w)) for 5 minutes to 15 minutes so that acetylene gas was dissolved in the substrate, which was performed immediately before supplying the substrate to the microbial electrolysis cell.

Particularly, the mixing ratio of acetylene gas to nitrogen may be 2% acetylene gas to 98% nitrogen (i.e., the mixing ratio of acetylene gas to nitrogen is 2:98 (w/w)).

### 4. Operation of microbial electrolysis cell

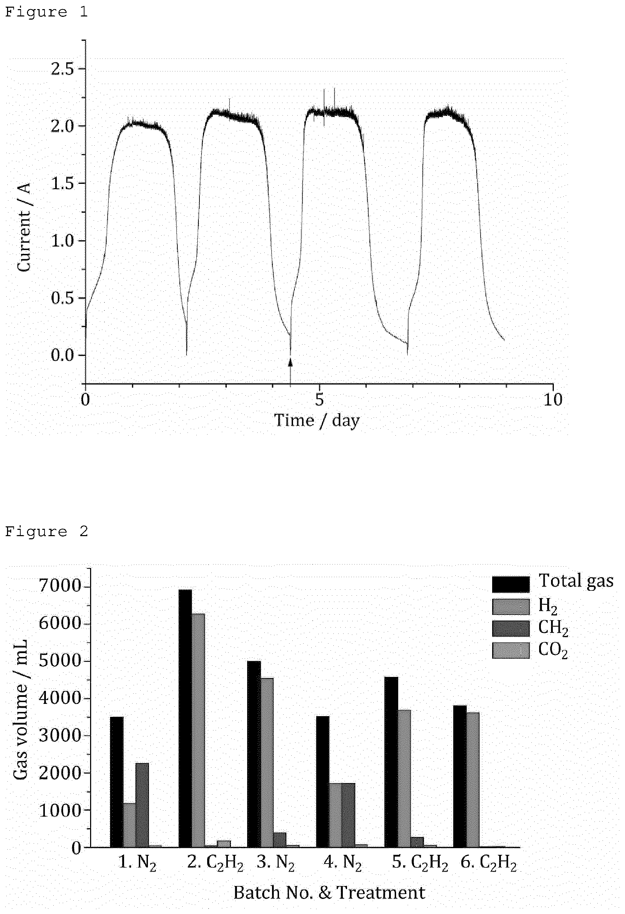

FIG. 1 is a graph showing the current generation pattern after inoculating a microbial electrolysis cell with microorganisms.

When a microbial electrolysis cell is inoculated with a substrate (i.e., microorganisms) obtained by treating activated sludge in wastewater with phosphoric acid, the generated current gradually increases, and after a certain period of time, a constant level of current is generated as shown in FIG. 1.

When the constant level of current was generated in the microbial electrolysis cell, only 50 mM PBS with 1 g/L sodium acetate was injected without activated sludge in a sewage treatment plant, after which current was measured by applying an external voltage of 0.8 V, and the generated gas was collected by water substitution by setting a measuring cylinder filled with water upside down in a water bath filled with water through a silicone tube. The amount of collected gas was determined by the volume collected using a measuring cylinder, and the volume of each gas was calculated by multiplying the total gas volume by the proportion of each component measured using gas chromatography.

### 5. Measurement of amount of methane generated depending on pretreatment

In various cases such as when a substrate in a microbial electrolysis cell was pretreated by aeration with an acetylene-mixed gas (hereinafter used interchangeably with "supply") or not pretreated and when an acetylene-mixed gas was resupplied or repeatedly supplied, the amounts of methane and hydrogen generated were measured.

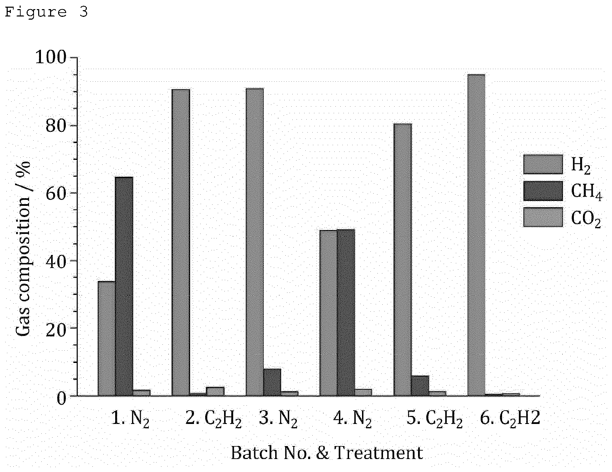

Table 1 below and FIGs. 2 and 3 show the amounts of gases generated by type in the various cases described above. Specifically, FIG. 2 is a graph comparing the amounts of gases generated by type in individual cases, and FIG. 3 is a graph comparing the proportions of gases generated by type in individual cases.

**[Table 1]**

| Unit: ml (%) | | | | |
|---|---|---|---|---|
| | Hydrogen | Methane | Carbon dioxide | Total gas amount |
| Batch No. 1 | 1181 (33.7) | 2261 (64.6) | 57.94 (1.7) | 3500 |
| Batch No. 2 | 6271 (90.6) | 57.09 (0.82) | 177.2 (2.56) | 6920 |
| Batch No. 3 | 4542 (90.8) | 397.5 (7.95) | 60.09 (1.2) | 5000 |
| Batch No. 4 | 1719 (48.8) | 1729 (49.1) | 71.54 (2.03) | 3520 |
| Batch No. 5 | 3688 (80.5) | 272.6 (5.95) | 59.1 (1.29) | 4580 |
| Batch No. 6 | 3620 (95.0) | 22.34 (0.59) | 27.64 (0.73) | 3810 |

In Batch No. 1, pretreatment with an acetylene/nitrogen-mixed gas was not performed. For Batch without pretreatment, the total amount of gases generated was 3500 ml, and respective amounts of hydrogen, methane, and carbon dioxide generated were 1181 ml, 2261 ml, and 57.94 ml, with methane gas being generated the most at 64.6% of the total (Batch No. 1 in Table 1 and FIGs. 2 and 3).

In Batch No. 2, pretreatment was performed with an acetylene/nitrogen-mixed gas (mixing ratio of acetylene gas to nitrogen of 2:98). As such, the total amount of gases generated was 6920 ml, and respective amounts of hydrogen, methane, and carbon dioxide generated were 6271 ml, 57.09 ml, and 177.2 ml, with hydrogen gas being generated the most at 90.6% (Batch No. 2 in Table 1 and FIGs. 2 and 3). Here, hydrogen production was increased to about 531% and methane gas generation was decreased to about 2.5%.

In Batch No. 3, the non-pretreated substrate was resupplied after Batch No. 2. As such, the total amount of gases generated was 5000 ml, and specifically, 4542 ml of hydrogen and 397.5 ml of methane were generated. Hydrogen gas was the most produced at 90.8%, but methane gas was also generated about 6.96 times more (Batch No. 3 in Table 1 and FIGs. 2 and 3).

In Batch No. 4, the non-pretreated substrate was supplied once more after Batch No. 3. As such, the total amount of gases generated was 3520 ml, and specifically, 1719 ml of hydrogen and 1729 ml of methane were generated, and the proportions of gases were 49.1% methane gas and 48.8% hydrogen gas (Batch No. 4 in Table 1 and FIGs. 2 and 3) .

In Batch No. 5, the pretreated substrate was supplied again after Batch No. 4. As such, the total amount of gases generated was 4580 ml. Specifically, the proportion of methane gas was decreased to 5.92%, and the generated amount thereof was 272.6 ml. Hydrogen gas was produced in an amount of 3688 ml, with a proportion of about 80.5% (Batch No. 5 in Table 1 and FIGs. 2 and 3).

In Batch No. 6, the pretreated substrate was repeatedly supplied after Batch No. 5. As such, the total amount of generated gases was 3810 ml. Specifically, methane gas generation was decreased to 0.59%, and the amount of methane gas generated was 22.3 ml (Batch No. 6 in Table 1 and FIGs. 2 and 3).

Taken together, when a substrate not pretreated with acetylene gas was supplied to a 10 L microbial electrolysis cell, methane gas generation was noticeable, but when a substrate pretreated with acetylene gas was supplied, the generation of methane gas was effectively suppressed. However, when the non-pretreated substrate was supplied thereto, methane gas was generated again, and the greatest amount of methane gas was generated when the non-pretreated substrate was repeatedly supplied.

In contrast, when the pretreated substrate was supplied again, the generation of methane gas was suppressed again, and when supply of the pretreated substrate was repeated, the generation of methane gas was decreased to 0.59%.

Meanwhile, in the embodiments described above, an electroactive biofilm was formed using a substrate in which activated sludge in a sewage treatment plant was mixed with PBS, but the present invention is not necessarily limited thereto. As an alternative to PBS, phosphoric acid may be used to adjust basic soju wastewater to a neutral pH of 7 and may serve as a phosphorus source.

As is apparent from the above description, according to an embodiment of the present invention, high-purity hydrogen can be produced by inhibiting methanogens through a pretreatment process of aerating a substrate with an acetylene-mixed gas, and the lifespan of the microbial electrolysis cell can be increased by preventing consumption of the hydrogen and substrate by methanogens.

Although some embodiments of the present invention have been disclosed for illustrative purposes, those skilled in the art will appreciate that various modifications and alterations are possible, without departing from the scope and spirit of the invention as disclosed in the accompanying claims.

## Claims

1. A method of producing hydrogen using a microbial electrolysis cell suppressing methane generation, comprising:
forming an electroactive biofilm on an anode;
aerating a liquid substrate with a mixed gas of acetylene gas and nitrogen at a predetermined ratio;
supplying the substrate in which acetylene gas is dissolved due to aeration; and
producing hydrogen at a cathode.

2. The method according to claim 1, wherein the liquid substrate is obtained by treating wastewater with phosphoric acid.

3. The method according to claim 2, wherein the wastewater is any one selected from among activated sludge in a sewage treatment plant and wastewater in a brewing house.

4. The method according to claim 1, wherein the mixed gas comprises acetylene gas and nitrogen mixed at a mixing ratio (w/w) of 2:98 to 3:97.

5. The method according to claim 1, wherein the substrate is aerated with the mixed gas for 5 minutes to 15 minutes.

6. A microbial electrolysis cell suppressing methane generation used in the method according to any one of claims 1 to 5.
